# EUROPEAN PATENT APPLICATION

(11) **EP 2 982 301 A1**
(43) Date of publication of application: **10.02.2016**
(21) Application number: 13881438.9
(22) Date of filing: 01.04.2013
(51) Int. Cl.: A61B 5/117

(54) **PALM VEIN IMAGE CAPTURE DEVICE**

(71) Applicant: Fujitsu Frontech Limited, Inagi-shi, Tokyo 206-8555 (JP)
(72) Inventor: YAMAZAKI, Kozo, Inagi-shi Tokyo 206-8555 (JP); IWAGUCHI, Isao, Inagi-shi Tokyo 206-8555 (JP); TANAKA, Hiroyuki, Inagi-shi Tokyo 206-8555 (JP); TSUJIKAWA, Akinori, Inagi-shi Tokyo 206-8555 (JP)
(74) Representative: Wilding, Frances Ward
(86) International application number: PCT/JP2013/059828
(87) International publication number: WO 2014/162388

(57) **Abstract**

A palm vein imaging apparatus is provided that is capable of being incorporated into a slim electronic device. A palm vein imaging apparatus (9) includes: an imaging system of an image sensor 18 and an imaging lens module (19); an illumination system of a plurality of illumination infrared ray LEDs 21 and a light guide body (22), the plurality of illumination infrared ray LEDs (21) being arranged in a circle around the imaging system; a distance-measuring system of a plurality of distance-measuring LEDs (24) and distance-measuring lens modules (25) located outside of the illumination system; a controlling system of a plurality of chip components (17) that include at least a CPU and a memory; a printed circuit board (16) that includes the imaging system, the illumination system, the distance-measuring system, and the controlling system on top and under surfaces thereof; a housing (13) that accommodates the printed circuit board together with the systems; a top cover (14) that covers the top opening of the housing; and a recess (15) at a portion of an inner top surface of the top cover that faces the imaging lens module, the recess (15) being thinner than a portion therearound, wherein the imaging lens module (19) is located in a manner such that an upper portion thereof is in the recess (15), and the apparatus has a thickness of 6mm or less.

## Description

### TECHNICAL FIELD

The invention relates to a palm vein imaging apparatus.

### BACKGROUND ART

Conventionally, imaging apparatuses that pick up an image of a predetermined range on an object by irradiating the object with uniform light have been widely used. Image processing systems that obtain sharp digital images for various applications by applying image processing to image data obtained by the imaging apparatus have also been widely put into practical use.

With recent developments in biometrics technology, various apparatuses have been provided that pick up an image of a feature of a living body with which an individual can be identified, e.g., finger prints or toe prints, a retina, a face, and blood vessels, so as to authenticate the individual by recognizing the feature of the living body.

Blood vessels in the palm or fingers and palm prints in particular provide a relatively large amount of personal feature data and are thus suitable for improving reliable personal authentication. The pattern of blood vessels (veins) is suitable for personal authentication because it does not change from the time a person is a fetus until death and is said to be unique to each person.

In regard to an imaging apparatus based on a conventional blood-vessel-image authentication technique that relies on the facts described above, a user moves her/his palm closer to the imaging apparatus for registration or authentication. The imaging apparatus emits near-infrared rays onto the palm. The imaging apparatus receives near-infrared rays reflected from the palm using an image sensor.

Hemoglobin within red blood cells flowing through a vein contains no oxygen. The hemoglobin (reduced hemoglobin) absorbs near-infrared rays of about 760 nanometers. Hence, when the palm is exposed to near-infrared rays, few rays are reflected from a portion with a vein, and the position of the vein can be recognized from the strength of the reflected near-infrared rays.

The user first registers her/his palm vein image data in a server and/or on an ID card using an imaging apparatus. Then, for personal authentication, the user causes the imaging apparatus to read vein image data of the palm. The user performs personal authentication by comparing the read vein image with the pattern of the vein in a registered vein image obtained using her/his ID.

Such an imaging apparatus may be downsized so that it can be incorporated into a device such as an ATM, wherein an image sensor and a plurality of light emitting elements therearound are mounted on a circuit board, and a ring-shaped light guide body illuminates an imaging area with light from the plurality of light emitting elements.

The imaging apparatus is configured in a manner such that an imaging optical unit is accommodated in the ring of the ring-shaped light guide body, and an image sensor is located at the bottom so that the image of reflected light from the imaging area illuminated by the ring-shaped light guide body can be received. Such an imaging apparatus, i.e., a downsized imaging apparatus with a light receiving system and a light emitting system mounted on the same board, is proposed. (See, for example, patent document 1.)

### PRIOR ART DOCUMENT

### PATENT DOCUMENT

Patent Document 1: Japanese Laid-open Patent Publication No. 2007-229360

### SUMMARY OF THE INVENTION

### PROBLEMS TO BE SOLVED BY THE INVENTION

Recently, personal authentication has also been requested to be performed for slim electronic devices such as laptop personal computers. As an example, a scheme has been put into practical use wherein fingerprint authentication is performed by touching an authentication panel with a fingertip.

Actual use of a fingerprint-authentication-based fingertip touch scheme is unexpectedly accompanied by a burdensome task. By contrast, the palm vein authentication described above is convenient owing to a scheme wherein a hand is held over an imaging apparatus.

However, according to patent document 1, an image sensor with a width of 640 dots is used. In general, an image sensor with a width of 640 dots leads to a chip size of about 6 × 7mm and a size of about 9x 9mm for a package mounted on a board.

According to the drawings of patent document 1 (e.g., a cross-sectional view of FIG. 1), with reference to width "a" of image sensor 30, the length and width of imaging apparatus 1 are "3.75a", and the height thereof is "3.0a". The length and width of imaging apparatus 1 is about 34x34mm, and the height thereof is about 27mm, where a=9mm.

In regard to slim electronic devices such as laptop personal computers, even a relatively thick one has a thickness of about 25mm, and a tabular portable electronic device consisting of only a touch-panel display screen has a thickness of 10mm or less. It is difficult to incorporate an imaging apparatus with a height of 27mm into such devices.

An object of the present invention is to provide a slim palm vein imaging apparatus that solves the conventional problem above and that is capable of being incorporated into a slim electronic device, e.g., a laptop personal computer.

### MEANS FOR SOLVING THE PROBLEMS

To solve the problem above, the palm vein imaging apparatus of the invention includes at least a tubular-frame-shaped housing, a printed circuit board horizontally held within the housing, an imaging system consisting of an image sensor and imaging lens module mounted on a top surface of the printed circuit board, and a top cover that covers a top opening of the housing so as to protect the imaging lens module of the imaging system. The top cover includes a recess at a portion of an inner top surface of the top cover that faces the imaging lens module, the recess being thinner than a portion therearound. The imaging lens module is located in a manner such that an upper portion thereof is in the recess.

In the palm vein imaging apparatus, a plurality of chip components forming a controlling system that includes at least a CPU and a memory are mounted on an under surface of the printed circuit board, and a gap is formed between a bottom surface of each of the chip components and a bottom opening surface of the housing, the gap being such that, when the housing is incorporated into an incorporation target apparatus, the chip components do not come into contact with an incorporation placement surface of the incorporation target apparatus.

In the palm vein imaging apparatus, a distance-measuring lens module consisting of a lens unit and an aperture unit may be mounted at each of the four corners of the printed circuit board. In this case, the lens unit of the distance-measuring lens module may be separated from the aperture unit and integrated with the top cover.

In the palm vein imaging apparatus, the recess may be cut into a circular shape or may have a truncated cone shape with a circular horizontal plane at the central portion and with an inclined plane extending from the circumference of the circular horizontal plane toward a surrounding thick portion. The height of the housing is 5mm or less. The thickness of the edge portions of the top cover is 1mm or less.

### EFFECT OF THE INVENTION

The present invention may provide a palm vein imaging apparatus capable of being incorporated into a slim electronic device, e.g., a laptop personal computer.

### BRIEF DESCRIPTION OF THE DRAWINGS

FIG. 1 (a) is a perspective view illustrating a laptop personal computer into which a palm vein imaging apparatus (imaging apparatus) in accordance with embodiment 1 of the present invention is incorporated;
FIG. 1(b) illustrates usage of the imaging apparatus;
FIG. 2 (a) is a sectional side view of an imaging apparatus in accordance with embodiment 1;
FIG. 2(b) is a cross-sectional view taken on line A-A' of FIG. 2 (a) (a diagram for arrangement of an image sensor and LEDs on a Pt board 16);
FIG. 2 (c) is an enlarged perspective view of a circular light guide body in FIG. 2(a);
FIG. 3 (a) is a sectional side view of a top cover of an imaging apparatus in accordance with embodiment 1;
FIG. 3(b) is a perspective view of the top cover in FIG. 3(a) viewed obliquely from below as indicated by arrow a;
FIG. 4 illustrates a more detailed configuration of a lens module of an imaging apparatus in accordance with embodiment 1, and details of an overall height of the imaging apparatus;
FIG. 5(a) is a sectional side view illustrating an imaging apparatus in accordance with embodiment 2;
FIG. 5 (b) is a perspective view of a top cover of the imaging apparatus in FIG. 5(a) as viewed obliquely from below within the apparatus;
FIGS. 5(C) and 5(d) simply illustrate components different from those in embodiment 1 for the purpose of comparison;
FIG. 6(a) is a sectional side view illustrating an imaging apparatus in accordance with embodiment 3; and
FIG. 6 (b) is a perspective view of a top cover of the imaging apparatus in FIG. 6(a) as viewed obliquely from below within the apparatus.

### EMBODIMENTS

The following will describe embodiments of the present invention in detail.

### [Embodiment 1]

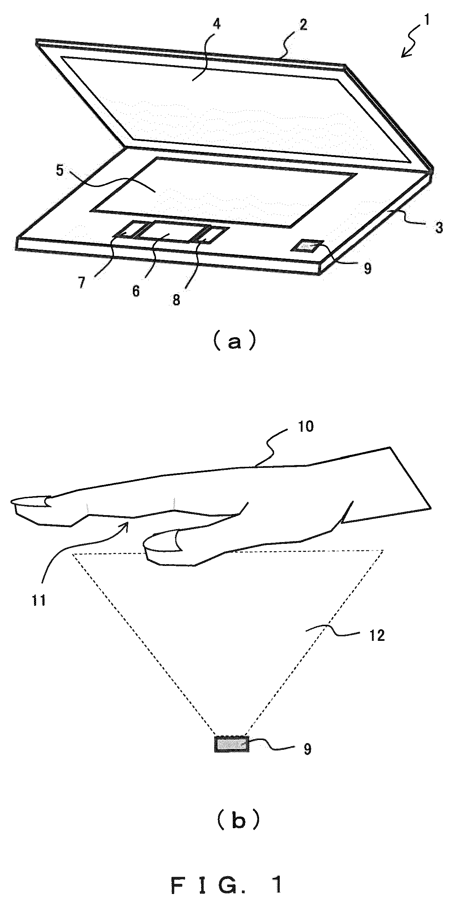

FIG. 1(a) is a perspective view illustrating a laptop personal computer into which a palm vein imaging apparatus (hereinafter simply referred to as an imaging apparatus) in accordance with embodiment 1 of the present invention is incorporated. FIG. 1(b) illustrates usage of the imaging apparatus.

As illustrated in Fig. 1(a), a laptop personal computer 1 includes a lid 2 and a main body 3, and a liquid crystal display apparatus 4 is formed on an inner surface of the lid 2. A keyboard 5 is formed that occupies a high proportion of a central portion of a top surface of the main body 3.

A touch-panel-type pointing device 6 is located on a central portion of a region below the keyboard 5, and a left click button 7 and a right click button 8 are respectively located to the left of and right of the touch-panel-type pointing device 6. An imaging apparatus 9 is located at a lower-right corner of the top surface of the main body 3.

The imaging apparatus 9 is an incorporable palm vein imaging apparatus. When a user 10 holds a palm 11 at a position about 50mm above the imaging apparatus 9, as depicted in FIG. 1(b), the imaging apparatus 9 emits near-infrared rays 12 with a particular wavelength, and an image sensor 18 of the imaging apparatus 9 reads a vein image of the palm 11 for personal authentication.

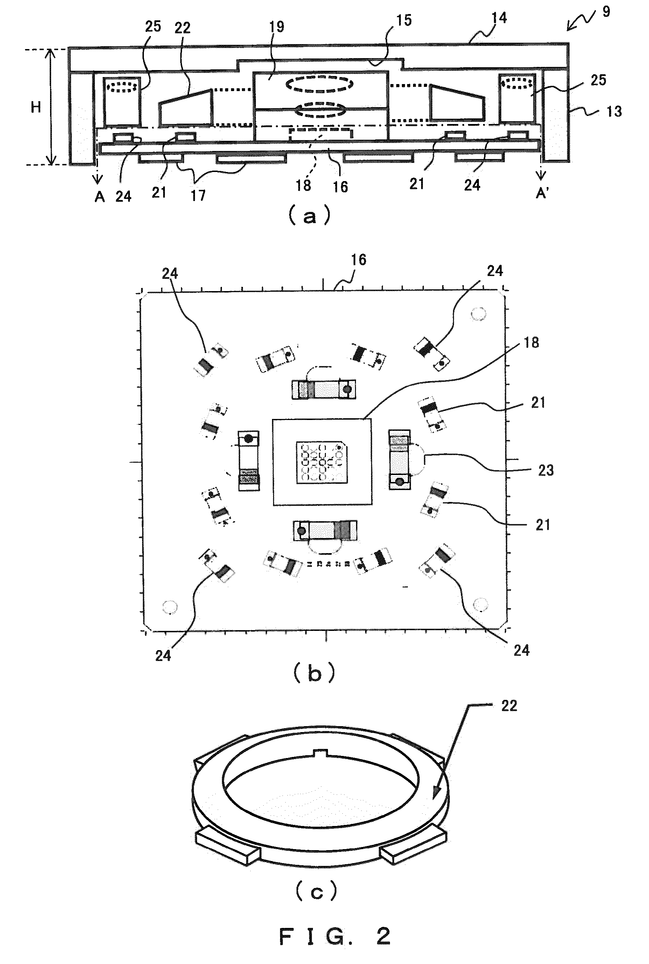

FIG. 2(a) is a sectional side view of the imaging apparatus 9. FIG. 2(b) is a plane view illustrating an arrangement of an image sensor 18 and LEDs on a printed board 16. FIG. 2(c) is an enlarged perspective view of a circular light guide body in FIG. 2(a).

As depicted in FIGS. 2 (a), 2(b), and 2(c), the imaging apparatus 9 includes a housing 13 virtually shaped like a square frame. The housing 13 includes an opening at the bottom thereof and a top cover 14 attached to an upper portion thereof. The top cover 14 has a certain strength for protecting internal members.

The top cover 14 is a transparent resin molding product. A recess 15 is formed on a central portion of the inner surface of the top cover 14 so as to make the entirety of the apparatus slim while maintaining a certain strength. The top cover 14 may be configured to further serve as a visible-light cut filter.

The printed board 16 is disposed and blocks the opening at the bottom of the housing 13. A plurality of chip components 17 that form a controlling g system such as a CPU or a memory are mounted on the bottom surface of the printed board 16.

An imaging system and an illumination system are disposed on the top surface of the printed board 16, the imaging system consisting of the image sensor 18 located at the center of the top surface of the printed board 16 and a lens module 19 located above the image sensor 18, and the illumination system consisting of a plurality of (in this example, eight) illumination LEDs 21 arranged in a circle around the image sensor 18 and a circular light guide body 22 located above the illumination LEDs 21.

The illumination LEDs 21 in the circular formation are divided into four blocks, and a light receiving element 23 is located at the center of each of the blocks. The light receiving element 23 is provided to control the light quantity of illumination LEDs 21 located to the right and left thereof.

In addition, a distance measurement system is disposed on the top surface of the printed board 16, the distance measurement system consisting of: a distance-measuring LED 24 for sensing a palm 11 and for measuring a distance to the palm 11; and a distance-measuring lens 25. These optical-system elements are constructed on the printed board 16, i.e., a single printed board, so as to downsize and make slim the entirety of the apparatus while decreasing the cost.

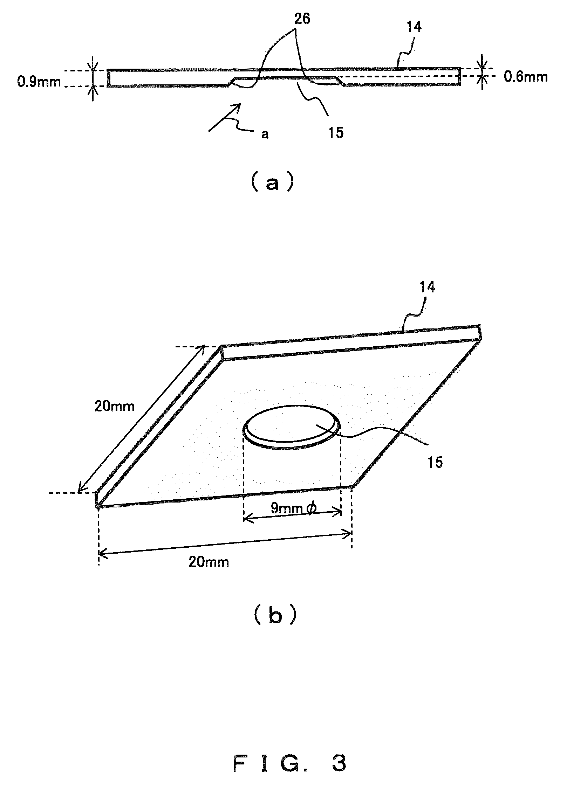

FIG. 3(a) is a sectional side view of the top cover 14. FIG. 3(b) is a perspective view of the top cover in FIG. 3(a) as viewed obliquely from below as indicated by arrow a. As depicted in FIGS. 3 (a) and 3(b), the length and width of the top cover 14 are each 20mm, and the thickness thereof is 0. 9mm. The recess 15 has a diameter ϕ=9.0mm and a surface thickness of 0.6mm.

As described above, the top cover 14 is a resin molding product. Accordingly, the diameter of an outer portion of an edge surface 26 of the recess 15 is made to be greater than the diameter of an inner portion thereof so that the top cover 14 can be readily removed from a die, i.e., so that a "hangover portion" is not formed.

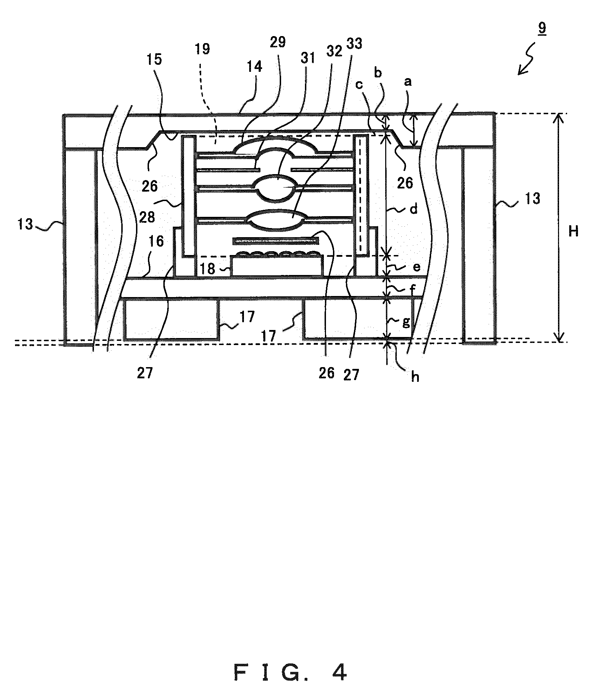

FIG. 4 illustrates a more detailed configuration of the lens module 19 of an imaging apparatus in accordance with embodiment 1, and details of an overall height of the imaging apparatus 9. In FIG. 4, like components are given like reference marks to those depicted in FIGS. 2 and 3.

Note that components located around the lens module 19 are not illustrated. In consideration of operability in an authentication operation, to shoot an image of the entirety of the palm 11 from a distance of, for example, about 50mm, the lens module 19 needs to have a wide-angle lens even if such a lens is small, and accordingly a set of three aspheric lenses such as that depicted in FIG. 4 is used.

The lens module 19 consists of a barrel 28 in which the set of lenses is stored, and a mount unit 27 that supports the barrel 28 and that connects the barrel 28 to the printed board 16. A first lens 29, a diaphragm 31, a second lens 32, a third lens 33, and a wavelength selection filter 26 are arranged in this order within the barrel 28, with the first lens 29 located at the highest position.

The top surface of the first lens 29 is close to the under surface of the recess 15 of the top cover 14, i.e., a top cover on which a void C for protecting a lens surface is formed. The wavelength selection filter 26 allows passage of only near-infrared ray wavelengths.

However, in a case where the top cover 14 is, as described above, configured to further serve as a visible-light cut filter, the wavelength selection filter 26 may be an IR cut filter that blocks infrared rays whose wavelength is longer than a near-infrared wavelength needed for the image sensor 18.

Irrespective of the top cover 14, a band-pass filter that blocks light other than light with a wavelength needed for the image sensor 18 may be used as a wavelength selection filter within the barrel 28.

In FIG. 4, a indicates the thickness of the top cover 14; b, a surface portion thickness of the recess 15; c, an interval between the under surface of the recess 15 and the upper portion of the lens module 19; d, the height of a top end of the lens after focus adjustment that is viewed from a sensor sensing surface; e, the thickness of the image sensor; f, the thickness of the printed board; g, the height of the chip component 17; h, a gap in a vertical direction between the bottom surface of the chip component 17 and a lower end of the housing 13.

Both the thickness a of the top cover 14 and the surface portion thickness b of the recess 15 need to be decreased as much as possible so as to make the apparatus slim, but the thicknesses depend on a purpose. The surface portion thickness b of the recess 15 is a limit thickness such that the recess 15 can be molded, and the thickness a of the edge portions is a limit thickness such that the strength of the top cover 14 can be maintained. The total length H of the entirety of the imaging apparatus 9 (see FIG. 1(a)) is H=b+c+d+e+g+g+h.

The actual dimensions (mm) are b=0.6, c=0.2, d=2.7, e=0.7, f=0.6, g=1.1, and h=0.1, and hence H=6.0mm.

In a case where the total height H of the entirety of the imaging apparatus 9 is, as described above, 6.0mm, the imaging apparatus 9 can be incorporated into a considerably thin electronic device. A protective plate is not provided at the bottom of the housing 13. However, owing to a gap of h=0.1mm in a vertical direction between the bottom surface of the chip component 17 and a lower end of the housing 13, a part on the imaging-apparatus-9 side and a part on the electronic-device side do not compete with each other for a space, and a failure does not occur.

### [Embodiment 2]

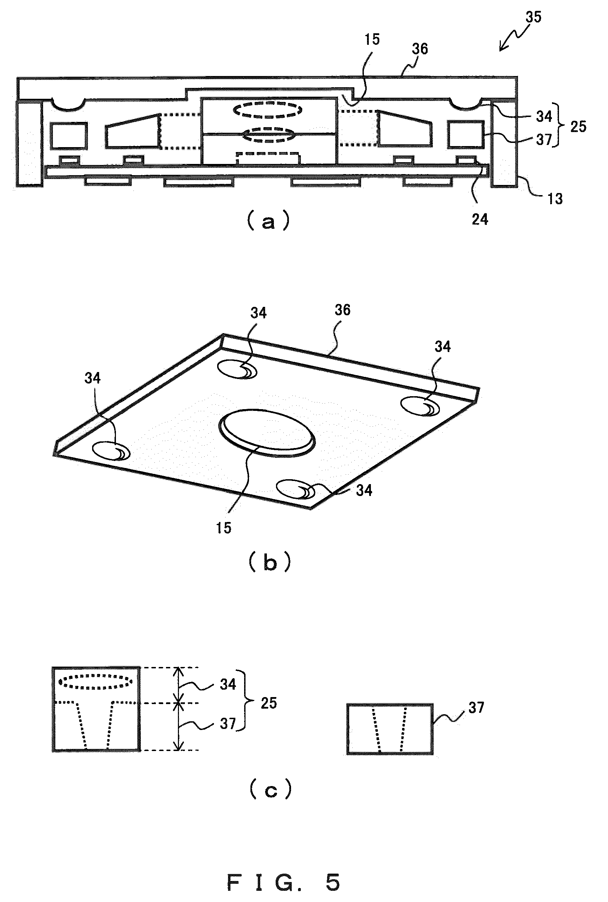

FIG. 5(a) is a sectional side view illustrating an imaging apparatus in accordance with embodiment 2. FIG. 5(b) is a perspective view of a top cover of the imaging apparatus in FIG. 5(a) as viewed obliquely from below within the apparatus. FIGS. 5(C) and 5 (d) simply illustrate components different from those in embodiment 1 for the purpose of comparison.

The components of an imaging apparatus 35 depicted in FIG. 5(a) are identical with those in embodiment 1 illustrated in FIG. 2(a), with some exceptions. Accordingly, in FIG. 5(a), only like components needed for descriptions are given like reference marks to those depicted in FIG. 2(a), with components different from those in FIG. 2 (a) given new reference marks.

As depicted in FIGS. 5(a) and 5(b), the imaging apparatus 35 in accordance with the present embodiment is configured in a manner such that the distance-measuring lens 25 depicted in FIG. 2 (a) is divided into upper and lower portions, and lens units 34 of the upper portion are formed at four corners of a top cover 36, and an aperture unit 37 of the lower portion is located on the housing-13 side.

As illustrated in FIG. 5(c), the distance-measuring lens 25 depicted in FIG. 2 (a) includes the aperture unit 37 and lens 34 integrated with each other. However, in the present embodiment, the aperture unit 37 is a functional unit for setting the diameter of a bore of the lens unit 34 through which light emitted from the distance-measuring LED 24 and to be incident on the lens unit 34 passes, and, as depicted in FIG. 5(c), the aperture unit 37 does not need to be integrated with the lens unit 34.

Accordingly, separating and locating, as depicted in FIG. 5(d), the aperture unit 37 on the housing-13 side with the lens units 34 formed, as depicted in FIGS. 5(a) and 5(b), at the four corners of the top cover 36 leads to an improved efficiency in manufacturing the lens units 34, thereby contributing to cost reduction.

### [Embodiment 3]

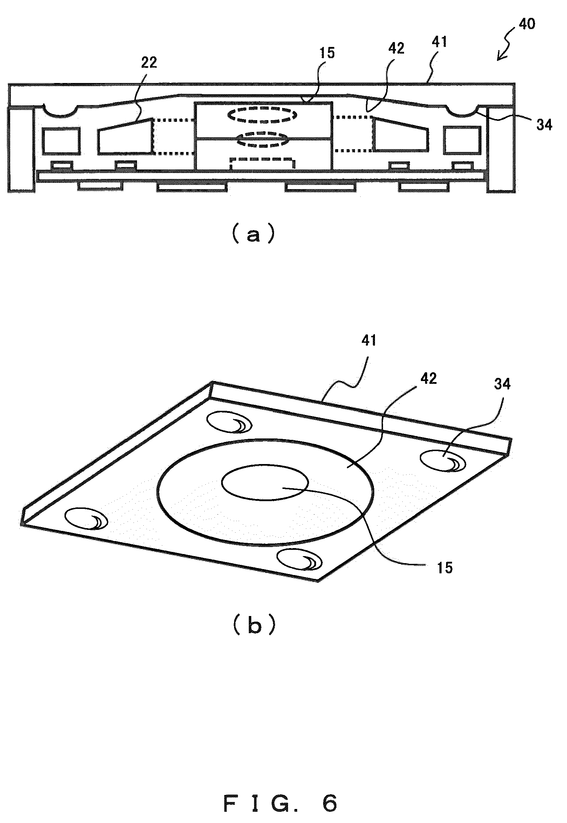

FIG. 6(a) is a sectional side view illustrating an imaging apparatus in accordance with embodiment 3. FIG. 6(b) is a perspective view of a top cover of the imaging apparatus in FIG. 6 (a) as viewed obliquely from below within the apparatus. The components of an imaging apparatus 40 depicted in FIG. 6 (a) are identical with those in embodiment 2 illustrated in FIG. 5(a) with some exceptions.

Accordingly, in FIG. 6(a), only like components needed for descriptions are given like reference marks to those depicted in FIG. 5(a), with components different from those in FIG. 5(a) given new reference marks.

As depicted in FIGS. 6(a) and 6(b), a top cover 41 of the imaging apparatus 40 in accordance with the present embodiment is shaped in a manner such that a plane ranging from an upper circumference of the recess 15 to an outside of a region through which passes illumination light emitted from the circular light guide body 22 becomes an inclined plane, unlike the recess 15 of the top cover 36 depicted in FIG. 5(a), which is dug in a circular shape as depicted in FIGS. 5(a) and 5(b).

Accordingly, the entirety of the recess assumes a shape of a truncated cone 42 that has an upper circumference of the original recess 15 as a top.

A dug recess such as that illustrated in FIG. 5 is typically difficult to mold because a material cannot easily spread and reach some positions within a forming die during a molding process. However, a recess having a truncated cone shape such as that depicted in FIG. 6 does not have a stair-like portion, and hence the material easily spreads throughout the forming die, thereby achieving the advantage of solving problems related to molding.

In the embodiments described above, the imaging apparatus has a thickness of 6mm. However, needless to say, an imaging apparatus having a thickness of 6mm or less may be achieved by, for example, making thinner the recess at the central portion of the inner surface of the top cover, the set of three aspheric lenses of the imaging lens module, the image sensor, and the chip components.

The present invention makes slim only a surface facing the imaging lens module while maintaining the strength of the top cover of the imaging apparatus so that the thickness of the imaging apparatus can be limited to 6mm or less, thereby achieving the advantage that the imaging apparatus can be readily incorporated into a slim electronic device.

Consequently, the imaging apparatus can be incorporated into more portable-electronic-device models so that personal authentication services can be provided to more users.

Various changes may be made to the embodiments described above without departing from the spirit thereof. In addition, although the imaging apparatus is incorporated into an electronic device in the embodiments described above, the imaging apparatus may be an external one independent of the electronic device.

### INDUSTRIAL APPLICABILITY

The present invention is applicable to a slim palm vein imaging apparatus that is capable of being incorporated into a slim electronic device.

### EXPLANATION OF THE CODES

- 1:: Laptop personal computer
- 2:: Lid
- 3:: Main body
- 4:: Liquid crystal display apparatus
- 5:: Keyboard
- 6:: Touch-panel-type pointing device
- 7:: Left click button
- 8:: Right click button
- 9:: Palm vein imaging apparatus (Imaging apparatus)
- 10:: User
- 11:: Palm
- 12:: Near-infrared rays
- 13:: Housing
- 14:: Top cover
- 15:: Recess
- 16:: Printed board
- 17:: Chip component
- 18:: Image sensor
- 19:: Lens module
- 21:: Illumination LED
- 22:: Circular light guide body
- 23:: Light receiving element
- 24:: Distance-measuring LED
- 25:: Distance-measuring lens
- 26:: Band-pass filter
- 27:: Mount
- 28:: Barrel
- 29:: First lens
- 31:: Diaphragm
- 32:: Second lens
- 33:: Third lens
- 34:: Lens unit
- 35:: Imaging apparatus
- 36:: Top cover
- 37:: Aperture unit
- 40:: Imaging apparatus
- 41:: Top cover
- 42:: Truncated cone

## Claims

1. A palm vein imaging apparatus comprising at least:
a tubular-frame-shaped housing;
a printed circuit board horizontally held within the housing;
an imaging system consisting of an image sensor and imaging lens module mounted on a top surface of the printed circuit board; and
a top cover that covers a top opening of the housing so as to protect the imaging lens module of the imaging system, wherein
the top cover includes a recess at a portion of an inner top surface of the top cover that faces the imaging lens module, the recess being thinner than a portion therearound, and
the imaging lens module is located in a manner such that an upper portion thereof is in the recess.

2. The palm vein imaging apparatus according to claim 1, wherein
a plurality of chip components forming a controlling system that includes at least a CPU and a memory are mounted on an under surface of the printed circuit board, and
a gap is formed between a bottom surface of each of the chip components and a bottom opening surface of the housing, the gap being such that, when the housing is incorporated into an incorporation target apparatus, the chip components do not come into contact with an incorporation placement surface of the incorporation target apparatus.

3. The palm vein imaging apparatus according to claim 1, wherein
a distance-measuring lens module consisting of a lens unit and an aperture unit is mounted at each of four corners of the printed circuit board.

4. The palm vein imaging apparatus according to claim 3, wherein
the lens unit of the distance-measuring lens module is separated from the aperture unit and integrated with the top cover.

5. The palm vein imaging apparatus according to claim 1, wherein
the recess is cut in a circular shape.

6. The palm vein imaging apparatus according to claim 1, wherein
the recess has a truncated cone shape with a circular horizontal plane at a central portion and with an inclined plane extending from a circumference of the circular horizontal plane toward a surrounding thick portion.

7. The palm vein imaging apparatus according to claim 1, wherein
the housing has a height of 5mm or less, and
edge portions of the top cover have a thickness of 1mm or less.
